# EUROPEAN PATENT SPECIFICATION

(11) **EP 3 852 678 B1**
(45) Date of publication and mention of the grant of the patent: **07.06.2023**
(21) Application number: 19773354.6
(22) Date of filing: 17.09.2019
(51) Int. Cl.: A61F 2/00

(54) **PENILE RING COMPRISING A SENSOR**
PENISRING MIT EINEM SENSOR
ANNEAU PÉNIEN COMPRENANT UN CAPTEUR

(30) Priority: 18.09.2018 DK PA201870607
(43) Date of publication of application: 28.07.2021
(73) Proprietor: Coloplast A/S, 3050 Humlebaek (DK)
(72) Inventor: GREENENKO, Alon, Cambridge CB4 1PQ (GB); COTTENDEN, David John, Melbourn SG8 6BJ (GB); VAN DEN BERGH, Roderick Marcus, Hitchin SG5 1PZ (GB); MAY, Rob, Cambridge CB6 2WW (GB)
(86) International application number: PCT/DK2019/050272
(87) International publication number: WO 2020/057705

(56) References cited:
- WO-A1-2018/137020
- WO-A1-2018/137634
- IT-A1- BS20 100 191
- US-A1- 2004 112 392
- US-A1- 2008 011 310

## Description

The invention relates to a penile ring.

### Summary of the Invention

The present disclosure provides aspects of penile ring according to the appended claims. The disclosure further describes a method of stopping a urine flow in urethra.

### Brief Description of the Drawings

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figure 1 illustrates an isometric view of an embodiment of a penile ring.
Figure 2 illustrates an embodiment of a penile ring in cross-section.
Figure 3 illustrates a cross-section of a penis.
Figure 4 illustrates an isometric view of an embodiment of a penile ring.
Figure 5 illustrates an isometric view of an embodiment of a penile ring.
Figure 6 illustrates a penile ring mounted on a penis.

### Detailed Description

Embodiments, and features of the various exemplary embodiments described in this application, may be combined with each other ("mixed and matched"), unless specifically noted otherwise.

In the following Detailed Description, reference is made to the accompanying drawings, which form a part hereof, and in which is shown by way of illustration specific embodiments in which the invention may be practiced. Because components of embodiments can be positioned in different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

The axial direction herein describes the direction of a longitudinal axis extending from a distal end to a proximal end of the penile ring. The transverse or radial direction is the direction perpendicular to the longitudinal direction/axial direction, which corresponds to the direction across the penile ring.

In the following, "closed configuration" of the penile ring addresses situations wherein the penile ring defines a closed ring-like structure, closed or secured by locking means. "Open configuration" of the penile ring addresses situations wherein the penile ring is open or not secured along the locking means.

Many people suffer from light incontinence. Stress incontinence means that a person may leak urine when sneezing, coughing, laughing, lifting something, changing position, or doing something that puts stress on a bladder. Urge incontinence is an urge to urinate that is so strong that a person cannot make it to the toilet in time. It also happens when a bladder squeezes when it should not. This can happen even when the amount of urine in the bladder is small. Overactive bladder is a type of urge incontinence. Where the amount of leaked urine in stress incontinence may be as small as few drops, the amount of leaked urine in urge incontinence may be larger and can be up to 180 ml a day.

Male urinary incontinence aiding systems are quite widespread. Widely used systems known as urisheaths normally comprise a roll-on sheath or body portion for enclosing the shaft of the penis, and a tip portion that is provided with a comparatively short discharge tube, which is connected to a urine collection bag via a tube and fastened to the leg of the user in some cases. Other solutions may include absorbent devices such as diapers. Alternatively, penile clamps may be used to be tightened around the penis to occlude the urethra. However, such clamps may be highly uncomfortable for the user and may induce tissue damage if worn for extended periods time.

US 2008/011310 discloses a penile ring having one compression pad.

WO 2018/137634 discloses a penile ring with three compression pads arranged spread over the inner surface.

WO 2018/137020, US 2004/112392 and IT BS20 100 191 disclose penile rings having one compression pad.

Incontinent users and health care professionals alike would welcome improved continence care devices which would be able to control stress incontinence and at the same time be discrete, comfortable and safe.

Embodiments relate to a penile ring adapted for closing the urethra to prevent stress and urge incontinence, comprising a ring-shaped body configured for surrounding a penis, the ring-shaped body comprising an upper (dorsal) part including a backstop support extending in an arc around the dorsal part of the penis, the ring-shaped body further comprising a lower(ventral) part including one or more compression pads, the compression pads being configured to extend inwards to apply a compression on the urethra, a sensor adapted for being in sensing contact with the urethra and being connectable to the ring-shaped body, so that upon sensing a urethral flow, the sensor generates a signal that triggers the compression pads to expand.

In embodiments, the ring-shaped body further comprises an actuator. In embodiments, the ring-shaped body further comprises a pump. In embodiments, the sensor sends a signal to the actuator, and the pump is connected to the actuator, which pump is adapted for inflating the compression pads upon actuation by the actuator.

The ring-shaped body of the penile ring is configured to enclose a portion of the shaft of a penis. In embodiments, the penile ring is worn at the root of the penis, being the proximal end of penis, closest to the body.

Having a backstop support around the dorsal part of the penis allows the expanding pads located in the ventral part of the ring-shaped body to apply controlled compression onto the penis and substantially occlude the urethral flow. In embodiments, the backstop support is sufficiently rigid to allow the moving pads to occlude the urethral flow. In embodiments, the ring-shaped body comprises two parts attachable together. The two parts may be combined to form a closed penile ring. In embodiments, the ring-shaped body comprises a hinged openable part, so the penile ring can be brought from open configuration to closed configuration and vice versa by operating the hinge. In embodiments, the ring-shaped body is provided with locking means securing the penile ring in closed configuration.

In embodiments, the ring-shaped body comprises soft inner linings, providing comfort towards the skin of the user. In embodiments, one or more features are provided on an inner surface of the ring-shaped body facing the penis to ensure that the ring-shaped body is sufficiently fixed in place during use.

In embodiments, the penile ring is provided with one or more compression pads. The compressing pads may be oriented to point towards the urethra, extending from the penile ring towards the urethra or towards a central axis of the penile ring. In embodiments, the penile ring is provided with three compression pads. The compression pads may be arranged side-by-side in the area closest to the urethra.

In embodiments, the sensor is provided on a connector attached or attachable to the ring-shaped body. In embodiments, the sensor is mounted on an elongated connector attachable or attached to the ring-shaped body allowing the sensor to be placed in a distance from the ring. In embodiments, the connector is flexible. The connector may be in the form of a band or strip. In embodiments, the connector comprises soft rubbery material, such as silicone or elastomer. In embodiments, the connector is detachable from the penile ring. In embodiments, the connector is in the form of a loop or ring attachable or attached to the ring-shaped body. In embodiments, the sensor is mounted on a condom-like strip attachable or attached to the ring-shaped body. In embodiments, the sensor may be integrated in the ring-shaped body.

In embodiments, the connector is in the form of a strip that is at least partly integrated with the inner surface of the penile ring. In embodiments, the strip may constitute at least a part of the lining of the ring-shaped body. The strip may comprise one or more sensors and the strip may be configured so that, when the penile ring is mounted on the penis, the sensors are positioned different places on the surface of the penis.

In embodiments, the sensor is wirelessly connected to the ring-shaped body.

In embodiments, the compression pads exert a pressure to the penis during occlusion, wherein the strain in the tunica albuginea is below 3-4 %. In embodiments, the compression pads apply a compression force to occlude the urethra by moving 2 - 10 mm towards the centre of the ring-shaped body.

In embodiments, the ring-shaped body comprises a means to operate the compression pads automatically and move them between a non-occluding and an occluding configuration. In embodiments, the ring-shaped body comprises control electronics assembled on a PCB (printed circuit board).

In embodiments, the ring-shaped body comprises an electric power supply. The electric power supply may be integrated in the penile ring such as in the upper part of the ring-shaped body. In embodiments, the ring-shaped body is configured to be connected to an external electric power supply.

In embodiments, the ring-shaped body is provided with an indicator for the electric power supply charge state. In embodiments, the ring-shaped body comprises an alarm, for example being activated when the electric power supply charge state is low.

In embodiments, the one or more sensors are configured to be positioned at the proximal side of the ring-shaped body. Having the sensor(s) placed upstream, the urine flow may be detected before the urine reaches the part of the urethra where the ring-shaped body is positioned and allow the one or more compression pads sufficient time to move to a configuration in which the urethral flow is occluded before the urine passes the ring-shaped body. Increasing the distance between the sensor and the ring-shaped body is beneficial in a sense that a larger distance provides longer warning times reducing the risk of urine leak because of the delay in operating the compression pads into occluding configuration. In one embodiment, one or more sensors are adapted for positioning under the scrotum in the perineal area. In embodiments, the sensor is joined to the ring-shaped body by a connector allowing the sensor to be placed in a required distance from the ring-shaped body. In embodiments, the sensor is adapted for positioning at the root of the penis, the connector extending from the ring-shaped body and towards the body of the user being upstream.

In embodiments, the sensor(s) is non-invasive in the sense that they are placed on the skin surface of the body.

In embodiments, the connector comprises a flexible strip, allowing the connector to follow the movements of the body. In embodiments, the connector comprises a rubbery substance such as silicone or elastomer.

Described detection methods include an electric impedance sensor whereby urine in the urethra can be detected through a change in electric impedance that appears when urine fills the urethra. Preferably, the electric impedance sensor comprises one or more electrodes in electrical contact with the tissue arranged in the ventral part of the penis, whereas the electrodes are separated by 0.1 to 20 mm. Preferably, the surface area of one or more sensor electrodes is between 0.1 to 100 square millimetres.

Preferably, the electrodes are attached to the penis by adhesive means. The electric impedance method has a low impact on perceived comfort for the user.

Preferably, the detection method includes a magnetic flow sensor whereby a flow of urine in the urethra can be detected through a change in electromotive force that appears when urine in the urethra flows in a magnetic field. In examples, the magnetic flow sensor comprises one or more electrodes in electrical contact with the tissue arranged in the ventral part of the penis, whereas the electrodes are separated by 0.1 to 20 mm. In examples, the surface area of one or more sensor electrodes is between 0.1 to 100 square millimetres. In examples, the electrodes are attached to the penis by adhesive means.

Preferably, the magnetic field is produced by one or more electromagnets arranged around a penis shaft. Preferably, the amplitude of the magnetic field is between 0.1 to 2 T. Preferably, the frequency of the magnetic field is between 0 to 100 kHz.

Doppler ultrasound may be employed to detect the urine flow as a shift in the frequency of ultrasound reflected from micro-bubbles in urethral flow. In examples, the detector is located in the perineal area thereby enabling the urine detection substantially as soon as it exits the bladder and enters the urethra. Preferably, the operating frequency of an ultrasound probe is between 10 kHz and 10 MHz.

In embodiments, the penile ring comprises an impedance spectroscopy sensor, a magnetic flowmeter; or an electromyographical based sensor.

In embodiments, the penile ring can apply a fixed pressure when worn most of the time. The pressure may be chosen to be high enough to occlude the urethra but low enough to avoid tissue damage and discomfort to the user but may prevent occasional leaks.

In embodiments, when needed, i.e. when a urine urge is detected, the compression pads can be configured to provide an extra compression to prevent a large volume urge/stress event. This force can be larger, i.e. above the tissue damage threshold, but still safe because the boost in the force is temporary.

Radial compression and urethra-focussed compression have higher mechanical complexity but better efficiency than linear compression so less pressure can be used to achieve an occlusion of urethra. A urethra-focussed compression with three compression pads arranged radially to the urethra or penis is more sensitive to correct placement of the ring but it does not need to be perfect, up to 10% deviation from correct position is acceptable.

It is also described a method of stopping a urine flow in the urethra comprising a penile ring adapted for closing the urethra to prevent stress or urge incontinence, comprising a ring-shaped body configured for surrounding a penis, the ring-shaped body comprising an upper (dorsal) part including a backstop support extending in an arc around the dorsal part of the penis, the ring-shaped body further comprising a lower (ventral) part including one or more compression pads, the compression pads being configured for expanding radially inwards to apply a compression on the urethra, mounting the ring-shaped body on a penis, placing a sensor in sensing contact with the urethra and being connected to the ring-shaped body upstream from the ring-shaped body, the sensor being configured to detect when a urine flow in urethra is initiated, a signal from the sensor triggering expansion of one or more compression pads into configuration in which the pads exert a pressure to the urethra so the urethra is occluded for passage of urine.

### Detailed Description of the Drawings

Embodiments, and features of the various exemplary embodiments described in this application, may be combined with each other ("mixed and matched"), unless specifically noted otherwise. The scope of the invention is defined by the appended claims.

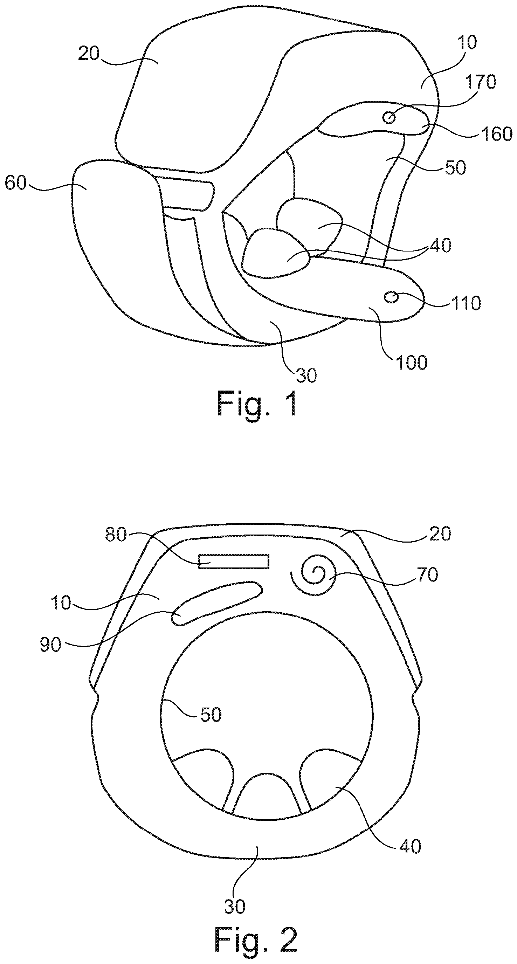

In Figure 1 is shown an embodiment of a penile ring, configured for surrounding a penis, comprising a ring-shaped body with an upper (dorsal) part (10) including a backstop (20) extending in an arc around the upper (dorsal) part (10). Furthermore, the ring-shaped body comprises a lower (ventral) part (30) including one or more compression pads (40). In the shown embodiment of Figure 1, the penile ring is provided with three compression pads (40), arranged at an inner (facing the penis) surface (50) of the ring-shaped body, at the lower part (30), the compression pads (40) being configured for expanding radially inwards to apply a compression to the urethra. The ring-shaped body comprises an openable part that allows the ring-shaped body to be opened or at least loosened to a wider diameter before application to a penis, thereby facilitating easy application. When the ring-shaped body is applied to surround the penis, the openable part is closed with locking means (60) and/or tightened to fit around the penis. The locking means (60) may be provided with steps allowing the ring-shaped body to be fitted to different sizes of penises. The backstop (20) is in the form of a fixed support, being less flexible/elastic than the lower part (30) of the ring-shaped body. The backstop (20) houses components being integrated in the upper part (10). A motor, PCB (80) and optionally an electrical power supply (90) are integrated in the upper part (10). The lower part (30) of the ring-shaped body may be flexible. The inner surface (50) of the ring-shaped body, the surface facing the penis, may be provided with soft inner linings to enhance the comfort for the user. In embodiments, the soft inner linings may comprise a soft silicone material or elastomer. Extending in axial direction is a connector (100) carrying a sensor (110). The connector (100) with the sensor (110) is extending upstream such that the sensor it is located between the ring-shaped body and the body of the user, preferably in the ventral side of the penis, closest to urethra. The sensor (110) detects a urine flow in the urethra, sends a signal that triggers an actuator or other means for expanding the compression pads, and the compression pads (40) are expanded to apply a pressure to the urethra, thereby stopping the urine flow. Having the sensor (110) upstream, it may be possible to stop the urine flow before it reaches the ring-shaped body and leakage can be avoided. A second connector (160) comprising a second sensor (170) may be present at the upper part (10) of the ring-shaped body.

In Figure 2 is shown a cross-section of an embodiment of a penile ring. At the upper part (10) is provided a backstop (20) and at the lower part (30) is provided compression pads (40) at the inner surface (50). Integrated in the upper part is a motor (70), PCB (80) and an electrical power supply (90).

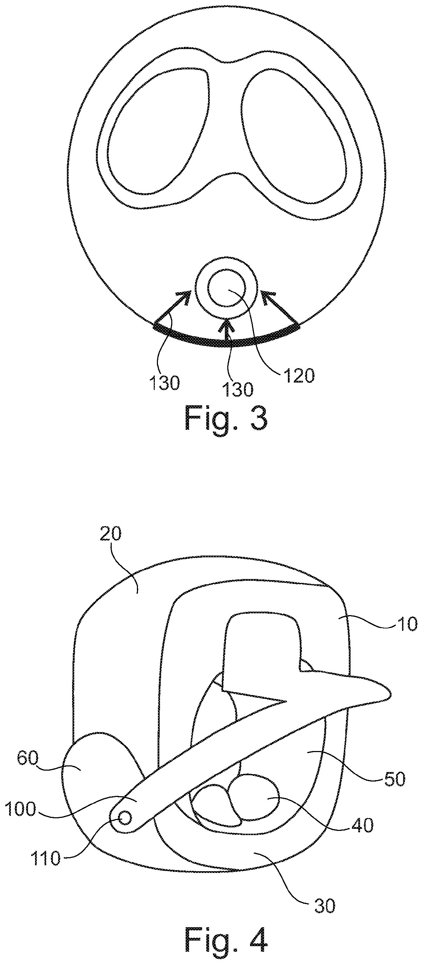

In Figure 3 is shown a cross-section of a penis. The urethra (120) is in the lower ventral portion of the penis and can be compressed and occluded by applying pressure to the penis at the lower portion. When a penile ring is arranged around the penis, such pressure may be applied by expanding one or more compression pads of the ring-shaped body. If pressure is applied from one point (such as if only one pressure pad is present/inflated), it will demand a higher pressure to occlude the urethra than if two or three compressing pads are present. Having three pressure pads, arranged as extending radially towards the urethra or centre of the penis, the urethra will be exposed to pressure from three sides as shown with arrows (130) in Figure 3, enabling the urethra to be occluded by applying less pressure than with one compression pad. The use of lower pressure enhances the comfort for the user as well as it enables that the ring can be used with this pressure for a prolonged period without causing tissue damage or discomfort for the user.

Figure 4 shows another embodiment of a penile ring, where the sensor is placed on an elongated connector extending upstream and in radial direction and facilitating that the sensor (110) is placed in a distance from the penile ring. This allows the sensor (110) to be placed for example in the perineal area.

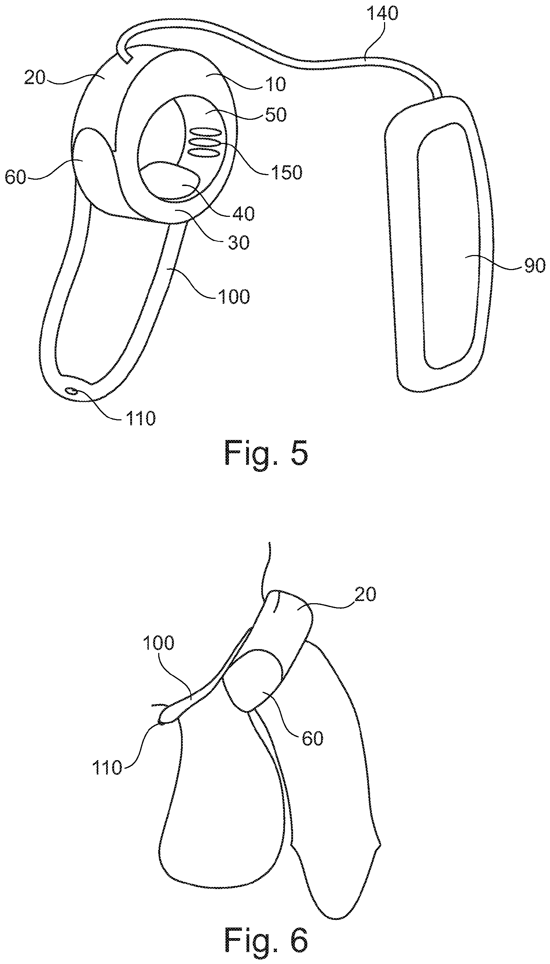

In Figure 5 is shown yet another embodiment of a penile ring, where the lower part (30) of the ring-shaped body is provided with a compression pad (40) and the inner surface (50) of the ring-shaped body is provided with features (150) for stabilizing the position of the ring on the penis. The features (150) may be in the form of raised ribs extending in axial direction as disclosed in Figure 5 but could be arranged in any suitable direction such as radial direction or a direction between radial and axial as well as they may be in the form of curved lines or dots. In embodiments, the features (150) are raised portions of the inner surface (50). In embodiments, the features (150) may be in the form of areas having high friction towards the skin. The penile ring is attached to an external electrical power supply (90) by a wire (140). A sensor (110) is connected to the ring-shaped body by a connector (100) in the form of a closed loop, the sensor (110) being arranged at the distal end of this connector (100). When the ring-shaped body is mounted on the penis, the connector (100) may be placed in the perineum area as shown in Figure 6. In this way, the sensor (110) will detect urine flow in the urethra when the urine passes in the perineal area and will send a signal to the ring-shaped body to trigger expansion of the compression pads (40) before the urine flow reaches the ring, and thereby leakage is avoided.

## Claims

1. A penile ring adapted for closing the urethra to prevent stress or urge incontinence, comprising
a. a ring-shaped body configured for surrounding a penis, the ring-shaped body comprising an upper dorsal part (10), a lower ventral part (30) and two or more compression pads ((40), the upper dorsal part (10) including a backstop (20) in the form of a fixed support, being less flexible and/or elastic than the lower ventral part (30) of the ring-shaped body and adapted to extend in an arc around the dorsal part of the penis,
b. a sensor (110) adapted for being in sensing contact with the urethra and being connectable to the ring-shaped body, so that upon sensing a urethral flow, the sensor is adapted to generate a signal that triggers the compression pads to expand; wherein the sensor (110) is joined to the ring-shaped body by a connector (100) allowing the sensor (110) to be placed in a distance from the ring,
**characterized in that** the lower ventral part (30) includes said two or more compression pads (40) arranged side-by-side and being configured for expanding inwards to apply a compression on the urethra.

2. The penile ring of claim 1, wherein the ring-shaped body comprises two parts attachable together.

3. The penile ring of claim 1, wherein the ring-shaped body comprises a hinged openable part.

4. The penile ring of claim 1, wherein one or more features for holding the ring-shaped body in place are provided on an inner surface of the ring-shaped body.

5. The penile ring of claim 1, wherein the penile ring comprises two or more sensors (110,170).

6. The penile ring of claim 1, wherein the ring-shaped body comprises an electrical power supply (90).

7. The penile ring of claim 1, wherein an external electrical power supply is connected or connectable to the ring-shaped body.

8. The penile ring of claim 1, wherein the penile ring is provided with an indicator for charge status of an electrical power supply.

9. The penile ring of claim 1, wherein a sensor is placed upstream, being positioned at the proximal side of the ring-shaped body.

10. The penile ring of claim 1, wherein the penile ring comprises an impedance spectroscopy sensor.

11. The penile ring of claim 1, wherein the penile ring comprises a magnetic flowmeter sensor.

12. The penile ring of claim 1, wherein the penile ring comprises an electromyographical based sensor.

## Patentansprüche

1. Penisring, der zum Verschließen der Urethra zur Prävention von Stress- oder Dranginkontinenz vorgesehen ist, umfassend:
a. einen ringförmigen Körper, der ausgestaltet ist, um einen Penis zu umgeben, wobei der ringförmige Körper ein oberes dorsales Teil (10), ein unteres ventrales Teil (30) und zwei oder mehr Kompressionskissen (40) umfasst, wobei das obere dorsale Teil (10) einen Backstop (20) in Form eines fixierten Trägers einschließt, der weniger flexibel und/oder elastisch als das untere ventrale Teil (30) des ringförmigen Körpers ist und vorgesehen ist, um sich im Bogen um den unteren dorsalen Teil des Penis zu erstrecken,
b. einen Sensor (110), der vorgesehen ist, um in Abfühlkontakt mit der Urethra zu sein, und mit dem ringförmigen Körper verbindbar ist, so dass der Sensor vorgesehen ist, um bei Abfühlen eines urethralen Flusses ein Signal zu generieren, welches das Expandieren der Kompressionskissen auslöst; wobei der Sensor (110) über einen Konnektor (100) mit dem ringförmigen Körper verbunden ist, wodurch der Sensor (110) in einem Abstand von dem Ring platziert werden kann,
**dadurch gekennzeichnet, dass** das untere ventrale Teil (30) die zwei oder mehr Kompressionskissen (40) einschließt, die nebeneinander angeordnet sind und ausgestaltet sind, um einwärts zu expandieren, um eine Kompression auf die Urethra auszuüben.

2. Penisring nach Anspruch 1, wobei der ringförmige Körper zwei Teile umfasst, die aneinander befestigbar sind.

3. Penisring nach Anspruch 1, wobei der ringförmige Körper ein klappbar zu öffnendes Teil umfasst.

4. Penisring nach Anspruch 1, wobei ein oder mehrere Merkmale, um den ringförmigen Körper in Position zu halten, auf einer Innenfläche des ringförmigen Körpers bereitgestellt werden.

5. Penisring nach Anspruch 1, wobei der Penisring zwei oder mehr Sensoren (110, 170) umfasst.

6. Penisring nach Anspruch 1, wobei der ringförmige Körper eine Stromzufuhr (90) umfasst.

7. Penisring nach Anspruch 1, wobei eine externe Stromversorgung mit dem ringförmigen Körper verbunden oder verbindbar ist.

8. Penisring nach Anspruch 1, wobei der Penisring mit einem Indikator für den Ladestatus einer Stromversorgung ausgestattet ist.

9. Penisring nach Anspruch 1, wobei ein Sensor stromaufwärts platziert ist und an der proximalen Seite des ringförmigen Körpers positioniert ist.

10. Penisring nach Anspruch 1, wobei der Penisring einen Impedanzspektroskopiesensor umfasst.

11. Penisring nach Anspruch 1, wobei der Penisring einen magnetischen Durchflussmesssensor umfasst.

12. Penisring nach Anspruch 1, wobei der Penisring einen Sensor auf elektromyographischer Basis umfasst.

## Revendications

1. Anneau pénien conçu pour fermer l'urètre afin de prévenir l'incontinence d'effort ou d'urgence, comprenant
a. un corps en forme d'anneau configuré pour entourer un pénis, le corps en forme d'anneau comprenant une partie dorsale supérieure (10), une partie ventrale inférieure (30) et deux ou plusieurs coussinets de compression (40), la partie dorsale supérieure (10) comprenant une butée arrière (20) sous la forme d'un support fixe, étant moins flexible et/ou élastique que la partie ventrale inférieure (30) du corps en forme d'anneau et conçue pour s'étendre en arc autour de la partie dorsale du pénis,
b. un capteur (110) conçu pour être en contact de détection avec l'urètre et pouvant être connecté au corps en forme d'anneau, de sorte que lors de la détection d'un écoulement urétral, le capteur soit conçu pour générer un signal qui déclenche l'expansion des coussinets de compression ; dans lequel le capteur (110) est connecté au corps en forme d'anneau par un connecteur (100) permettant au capteur (110) d'être placé à une certaine distance de l'anneau,
**caractérisé en ce que** la partie ventrale inférieure (30) comprend lesdits deux ou plusieurs coussinets de compression (40) disposés côte à côte et étant configurés pour se dilater vers l'intérieur afin d'appliquer une compression sur l'urètre.

2. Anneau pénien selon la revendication 1, dans lequel le corps en forme d'anneau comprend deux parties pouvant être attachées ensemble.

3. Anneau pénien selon la revendication 1, dans lequel le corps en forme d'anneau comprend une partie articulée pouvant être ouverte.

4. Anneau pénien selon la revendication 1, dans lequel une ou plusieurs caractéristiques pour maintenir le corps en forme d'anneau en place sont prévues sur une surface interne du corps en forme d'anneau.

5. Anneau pénien selon la revendication 1, l'anneau pénien comprenant deux ou plusieurs capteurs (110, 170).

6. Anneau pénien selon la revendication 1, dans lequel le corps en forme d'anneau comprend une alimentation électrique (90).

7. Anneau pénien selon la revendication 1, dans lequel une alimentation électrique externe est connectée ou peut être connectée au corps en forme d'anneau.

8. Anneau pénien selon la revendication 1, l'anneau pénien étant pourvu d'un indicateur d'état de charge d'une alimentation électrique.

9. Anneau pénien selon la revendication 1, dans lequel un capteur est placé en amont, étant positionné au niveau du côté proximal du corps en forme d'anneau.

10. Anneau pénien de la revendication 1, l'anneau pénien comprenant un capteur de spectroscopie d'impédance.

11. Anneau pénien selon la revendication 1, l'anneau pénien comprenant un capteur de débitmètre magnétique.

12. Anneau pénien selon la revendication 1, l'anneau pénien comprenant un capteur basé sur l'électromyographie.
